# EUROPEAN PATENT APPLICATION

(11) **EP 2 989 968 A1**
(43) Date of publication of application: **02.03.2016**
(21) Application number: 15174277.2
(22) Date of filing: 29.06.2015
(51) Int. Cl.: A61B 5/00, A61B 8/00

(54) **OPTOACOUSTIC WAVE DETECTOR AND OPTOACOUSTIC IMAGING DEVICE**

(30) Priority: 28.08.2014 JP 2014174288
(71) Applicant: PreXion Corporation, Chiyoda-ku Tokyo 101-0041 (JP)
(72) Inventor: SHIGETA, Yusuke, Chiyoda-ku, Tokyo 101-0041 (JP)
(74) Representative: Grünecker Patent- und Rechtsanwälte PartG mbB

(57) **Abstract**

An optoacoustic imaging device (100) has an optoacoustic detector (20), a cross-sectional image generator (305, 306, 307, 311), and an image former (305, 306, 307, 311). The optoacoustic detector (20) has a light source (24) for irradiating a light absorber (P) inside a tested object (150) with light, and has an acoustoelectric converter (25). The acoustoelectric converter (25) detects, and generates a detection signal of, an optoacoustic wave generated by the light absorber (P). The acoustoelectric converter (25) has a plurality of detecting elements (251) arrayed one-dimensionally, and the light source (24) includes light-emitting elements (241) arranged at least between adjacent ones of the detecting elements (251). The cross-sectional image generator (305, 306, 307, 311) generates a cross-sectional image of the tested object (150) based on the detection signal, and the image former (305, 306, 307, 311) forms an optoacoustic image based on the cross-sectional image.

## Description

This application is based on Japanese Patent Application No. 2014-174288 filed on August 28, 2014, the contents of which are hereby incorporated by reference.

### BACKGROUND OF THE INVENTION

### 1. Field of the Invention

The present invention relates to optoacoustic wave detectors and optoacoustic imaging devices.

### 2. Description of Related Art

In recent years, there have been developed optoacoustic imaging devices that allow non-destructive inspection of a particular object inside a living body (see Japanese patent application published No. 2010-12295). An optoacoustic imaging device irradiates a living body with light of a predetermined wavelength and detects an optoacoustic wave which is an elastic wave emitted back from a particular object (light absorber) inside the living body when this object absorbs the light. The optoacoustic imaging device then generates a cross-sectional image based on the result of detection of the optoacoustic wave, and thereby generates an optoacoustic image showing the light absorber inside the living body which is present in a region right under an optoacoustic wave detector.

Typically used as a light source of the light with which to irradiate the living body is, for example, a solid-state laser. This permits irradiation with high-output laser light, hence generation of an intense optoacoustic wave, and hence formation of a sharp optoacoustic image. However, using a laser as a light source requires that an optoacoustic imaging device be furnished with a laser light generating device. This leads to an increased device size and a complicated device design, and makes cost reduction difficult.

As a remedy, for example according to Japanese patent application published No. 2010-12295, in an applicator that irradiates a tested object with light and detects an optoacoustic wave, optical fiber ends and acoustoelectric conversion elements are arrayed alternately. Light emitted from a light-emitting element and generated outside the applicator is guided through optical fibers to be shone on the tested object.

However, using a light-emitting element or the like as an external light source as disclosed in Japanese patent application published No. 2010-12295 results in the emitted light having a beam spread angle wider than laser light. This makes it difficult to guide light generated outside the applicator through optical fibers. An attempt to guide light through optical fibers ends in, for example, an increased device size. Moreover, Japanese patent application published No. 2010-12295 does not specifically disclose how to guide light when a light-emitting element is used as an external light source.

### SUMMARY OF THE INVENTION

Devised against the background discussed above, the present invention aims to provide an optoacoustic wave detector and an optoacoustic imaging device that can detect an optoacoustic wave sufficiently intense to generate an cross-sectional image with a simple structure employing a light source of a comparatively low output.

To achieve the above object, according to one aspect of the present invention, an optoacoustic wave detector includes a light source and an acoustoelectric converter. The light source irradiates a light absorber inside a tested object with light. The acoustoelectric converter detects an optoacoustic wave generated by the light absorber to generate a detection signal based on the result of detection of the optoacoustic wave. The acoustoelectric converter has a plurality of detecting elements arrayed one-dimensionally. The light source includes light-emitting elements arranged at least between adjacent ones of the detecting elements.

According to another aspect of the present invention, an optoacoustic imaging device includes an optoacoustic wave detector as described above, a cross-sectional image generator, and an image former. The cross-sectional image generator generates a cross-sectional image of the tested object based on the detection signal. The image former forms an optoacoustic image based on the cross-sectional image.

Further features and advantages of the present invention will become apparent from the description of embodiments given below

### BRIEF DESCRIPTION OF THE DRAWINGS

Fig. 1 is a perspective external view of an optoacoustic imaging device;
Fig. 2 is a block diagram showing an example of the configuration of an optoacoustic imaging device;
Fig. 3A is a schematic sectional view of an ultrasonic probe as seen from a side;
Fig. 3B is a schematic sectional view of an ultrasonic probe as seen from another side;
Fig. 4A is a schematic perspective view showing an example of the structure of an optoacoustic module according to a first embodiment;
Fig. 4B is a schematic side view showing an example of the structure of an optoacoustic module according to the first embodiment;
Fig. 4C is a schematic side view showing another example of the structure of an optoacoustic module according to the first embodiment;
Fig. 5A is a schematic perspective view showing an example of the structure of an optoacoustic module according to a second embodiment;
Fig. 5B is a schematic top view showing an example of the structure of an optoacoustic module according to the second embodiment;
Fig. 5C is a schematic top view showing another example of the structure of an optoacoustic module according to the second embodiment;
Fig. 6A is a schematic perspective view showing an example of the structure of an optoacoustic module according to a third embodiment;
Fig. 6B is a schematic side view showing an example of the structure of an optoacoustic module according to the third embodiment;
Fig. 7A is a schematic perspective view showing an example of the structure of an optoacoustic module according to a fourth embodiment;
Fig. 7B is a schematic side view showing an example of the structure of an optoacoustic module according to the fourth embodiment;
Fig. 8A is a schematic perspective view showing an example of the structure of an optoacoustic module according to a fifth embodiment; and
Fig. 8B is a schematic side view showing an example of the structure of an optoacoustic module according to the fifth embodiment.

### DETAILED DESCRIPTION OF PREFERRED EMBODIMENTS

Hereinafter, embodiments of the present invention will be described with reference to the accompanying drawings.

### First Embodiment

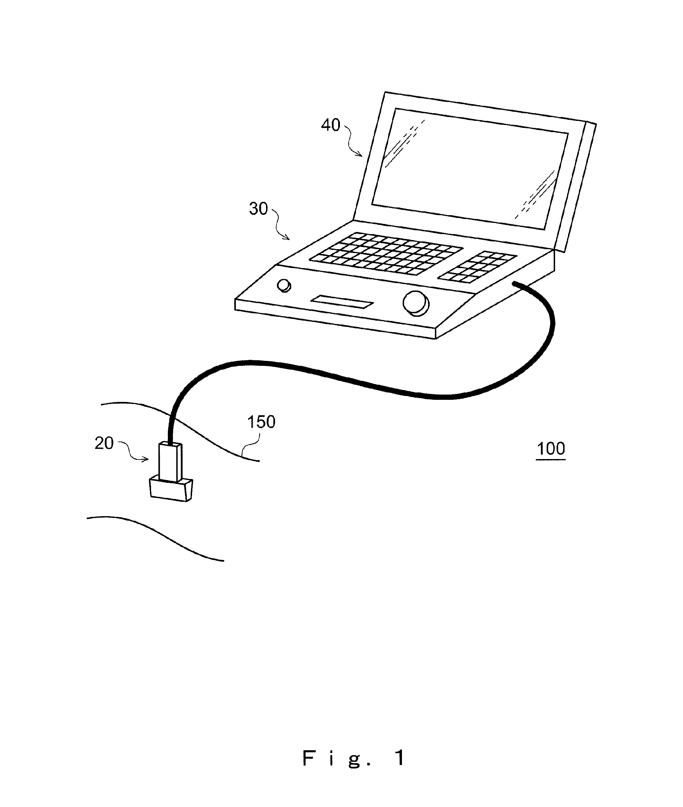

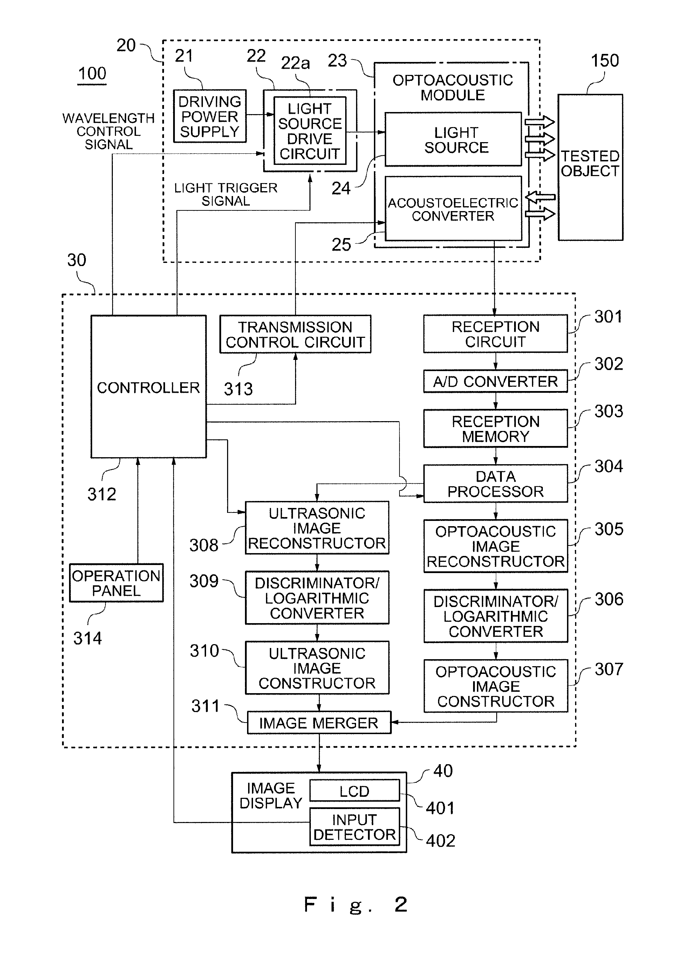

Fig. 1 is a perspective external view of an optoacoustic imaging device 100. Fig. 2 is a block diagram showing an example of the configuration of the optoacoustic imaging device 100. As shown in Fig. 1, the optoacoustic imaging device 100 includes an ultrasonic probe 20 for acquiring cross-sectional image information from inside a tested object 150, an image generator 30, and an image display 40.

The ultrasonic probe 20 irradiates the tested object 150, which is a living body for instance, with light and detects an optoacoustic wave, which is an elastic wave, generated inside the tested object 150 as a result. The ultrasonic probe 20 transmits an ultrasonic wave into the tested object 150 and detects an elastic wave (ultrasonic wave) reflected inside the tested object 150.

As shown in Fig. 2, the ultrasonic probe 20 includes a driving power supply 21, a light source driver 22, and an optoacoustic module 23. The driving power supply 21 supplies electric power to the light source driver 22. The light source driver 22 is configured to include a light source drive circuit 22a which drives and controls a light source 24. The ultrasonic probe 20 may further include a lens member for converging the light emitted from the light source 24, a light guide (e.g., a light guide plate of acrylic resin, an optical fiber) for guiding the light converged by the lens member to the tested object 150, etc.

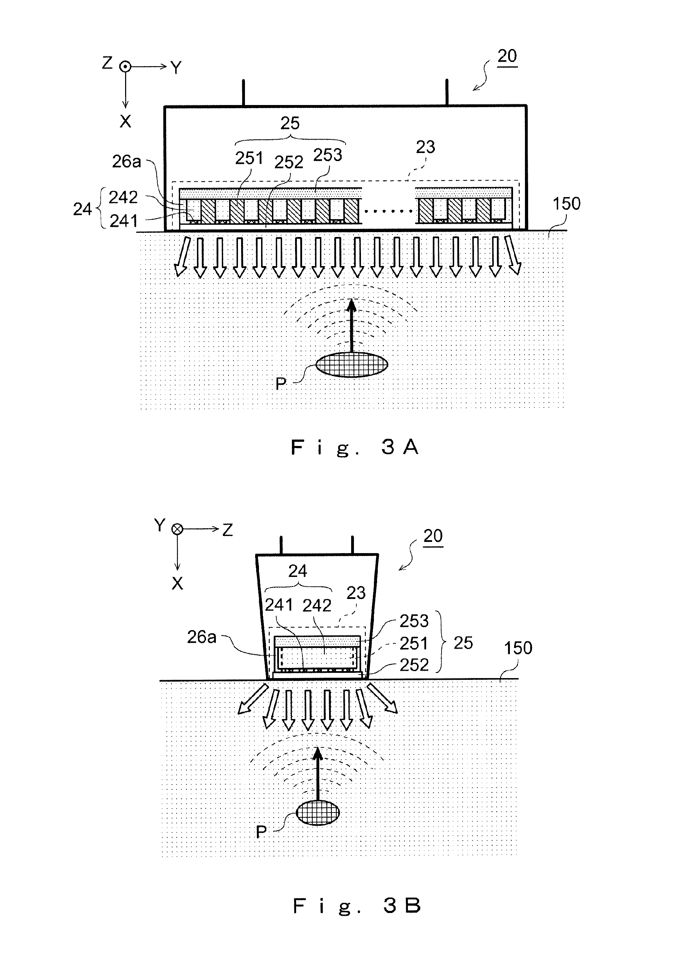

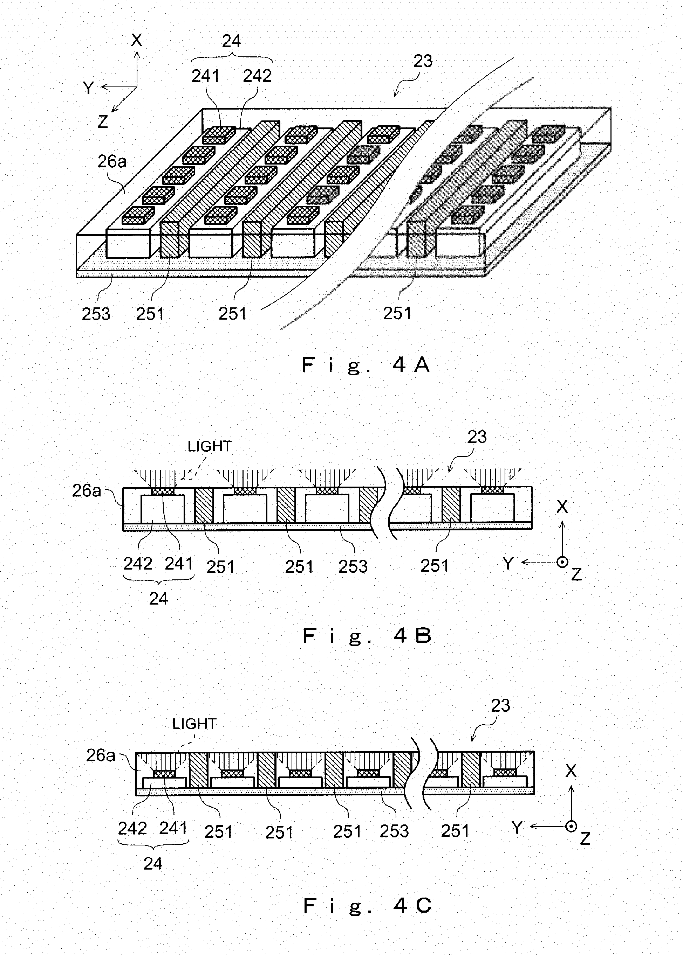

Fig. 3A is a schematic sectional view of the ultrasonic probe 20 as seen from a side. Fig. 3B is a schematic sectional view of the ultrasonic probe 20 as seen from another side. Fig. 4A is a schematic perspective view showing an example of the structure of the optoacoustic module 23 according to a first embodiment of the present invention. Fig. 4B is a schematic side view showing an example of the structure of the optoacoustic module 23 according to the first embodiment. While Fig. 3A and 3B show the irradiating direction and the ultrasonic wave detecting surface pointing downward, Figs. 4A and 4B show them pointing upward for the sake of easy understanding of the structure of the optoacoustic module 23. That is, Figs. 4A and 4B are views rotated 180 degrees about a Z-direction axis relative to the views of Figs. 3A and 3B. A similar positional relationship applies to Figs. 4C to 8B, which will be referred to later.

As shown in Fig. 3A and 3B, when optoacoustic imaging or ultrasonic imaging is performed by use of the ultrasonic probe 20, the optoacoustic module 23 is placed in contact with the surface of the tested object 150. Here, between the tested object 150 and the optoacoustic module 23, a gel layer (unillustrated) is provided to reduce the difference in acoustic impedance between them. The gel layer acts as an acoustic coupling member which serves to propagate the ultrasonic wave emitted from the ultrasonic probe 20 efficiently into the tested object 150. The gel layer also serves to propagate the ultrasonic wave (including an optoacoustic wave) from inside the tested object 150 efficiently to the ultrasonic probe 20.

The optoacoustic module 23 is configured to include a light source 24, an acoustoelectric converter 25, and a sealing layer 26a.

The light source 24 is an LED light source which irradiates the interior (in particular, a light-absorber P) of the tested object 150 with light. The light source 24 is configured to include a plurality of LED elements 241 and a plurality of substrates 242.

The lighting of the LED elements 241 is controlled by the light source drive circuit 22a. The light emitted from the LED elements 241 is shone on the tested object 150. The LED elements 241 are an LED light source which is driven to emit pulsating light. There is no particular limitation on the PRF (pulse repetition frequency) of the pulsating light; it can be, for example, 1,000 pulses per second. As shown in Figs. 3A and 3B, the pulsating light is shone into the tested object 150 while being diffused, and is absorbed by a light absorber P (living tissue) inside the tested object 150. As the light absorber P absorbs light, adiabatic expansion occurs, whereby an optoacoustic wave (ultrasonic wave), which is an elastic wave, is generated. The optoacoustic wave propagates inside the tested object 150, and is converted into a voltage signal by the acoustoelectric converter 25.

By using an LED light source as the light source 24 in this way, it is possible to make the light source 24 compact, and to irradiate the tested object with pulsating light having a high emission frequency with a simple structure. It is thus possible to acquire more cross-sectional image information per unit time (e.g., the period required to update the display on an LCD 401, which will be described later), and thus to obtain an optoacoustic image with higher sharpness and higher definition.

The LED elements 241 may be composed of LED light sources with an equal emission wavelength, or may be composed of a plurality of types of LED light sources with different emission wavelengths. The emission wavelength of the LED elements 241 can be set at a wavelength at which the test target (light absorber P) exhibits a high absorptance. For example, in a case where the test target is oxidized hemoglobin in blood, the emission wavelength can be set at 760 nm at which oxidized hemoglobin exhibits a high absorptance. For another example, in a case where the test target is reduced hemoglobin in blood, the emission wavelength can be set at 850 nm at which reduced hemoglobin exhibits a high absorptance. For example, when the tested object 150 is irradiated with pulsating light with a wavelength of 760 nm, the light is absorbed by the oxidized hemoglobin in the blood contained in arteries, tumors, etc. inside the tested object 150, and an optoacoustic wave is generated as a result. Based on the optoacoustic wave, an optoacoustic image constructor 307, which will be described later, generates an optoacoustic image showing arteries, tumors, etc.

The substrates 242 are, for example, Al substrates, and have the LED elements 241 mounted on them. This, however, is not meant as any limitation; the substrates 242 may instead by printed circuit boards with a wiring pattern, or metal substrates of any other electrically conductive material (e.g., Cu substrates).

The acoustoelectric converter 25 is configured to include a plurality of (e.g., 128) ultrasonic oscillating elements 251, an acoustic coupling layer (unillustrated), an acoustic lens 252, and a backing member 253.

The ultrasonic oscillating elements 251 are piezoelectric elements which are arrayed one-dimensionally in the Y direction. When a voltage is applied to the ultrasonic oscillating elements 251, these oscillate and generate an ultrasonic wave; when vibration (an ultrasonic wave) is applied to the ultrasonic oscillating elements 251, these generate a voltage. The ultrasonic oscillating elements 251, on one hand, generate an ultrasonic wave and propagate it into the tested object 150 and, on the other hand, detect the ultrasonic wave reflected inside the tested object 150 and generate a voltage signal. Thus, by use of the ultrasonic probe 20, not only optoacoustic imaging but also ultrasonic imaging can be performed.

The ultrasonic oscillating elements 251 are arrayed on the backing member 253 (e.g., in Figs. 3A and 3B, on the lower principal surface) so as to alternate, in the Y direction, with the substrates 242 on which the LED elements 241 are mounted. There is no particular limitation on how to array the ultrasonic oscillating elements 251 and the substrates 242; the minimum requirement is that substrates 242 having LED elements 241 mounted on them be arranged at least between adjacent ultrasonic oscillating elements 251. It is preferable that, as shown in Figs. 3A and 3b, a substrate 242 having LED elements 241 mounted on it be arranged at each end of the array of the ultrasonic oscillating elements 251.

With this structure, it is possible to simplify the structure of the optoacoustic module 23, and to enhance the efficiency of use of the light emitted from the LED elements 241. It is also possible to irradiate the tested object 150 with the light emitted from the LED elements 241 efficiently from a position very close to the ultrasonic oscillating elements 251. It is thus possible to generate an intense optoacoustic wave at the light absorber P inside the tested object 150, and to increase the detection intensity of the optoacoustic wave. Hence, it is possible, by use of a light source 24 of a comparatively low output, to detect an optoacoustic wave sufficiently intense to generate a cross-sectional image.

As shown in Figs. 3A and 3B, on the tested object 150 side surface (the surface pointing in the X direction) of the ultrasonic oscillating elements 251, there are provided an acoustic coupling layer and an acoustic lens 252 in this order. In Figs. 4A and 4B, for the sake of easy understanding of the structure, the acoustic lens 252 is omitted. The acoustic lens 252 is an acoustic converging member, and serves to converge the ultrasonic wave emitted from the ultrasonic oscillating elements 251. The acoustic impedance between the ultrasonic oscillating elements 251 and the acoustic lens 252 is adjusted by the acoustic coupling layer. The backing member 253 suppresses rearward (e.g., in Figs. 3A and 3B, upward of the backing member 253) propagation of the ultrasonic wave generated by the ultrasonic oscillating elements 251.

The height in the X direction of the light emission position of the LED elements 241 and of the top surface (the surface pointing in the X direction) of the ultrasonic oscillating elements 251 may be equal as in Fig. 4B, or may be different. Fig. 4C is a schematic side view showing another example of the structure of the optoacoustic module 23 according to the first embodiment. For example, as shown in Fig. 4C, the light emission position of the LED elements 241 may be lower in the X direction than the top surface of the ultrasonic oscillating elements 251. With this structure, it is easy to irradiate a region inside the tested object 150 close to the detecting elements (e.g., in Figs. 3A and 3B, a region right under the ultrasonic probe 20) with the light emitted from the LED elements 241 with a comparatively wide beam spread angle. It is thus possible to further increase the intensity of the optoacoustic wave generated at a position close to the ultrasonic oscillating elements 251.

The sealing layer 26a is formed of, for example, silicone, and seals in the plurality of LED elements 241. The material of the sealing layer 26a can be any transparent or translucent material that transmits the light emitted from the plurality of LED elements 241. The sealing layer 26a may instead be formed of, for example, glass, any other transparent or translucent resin material, or a transparent or translucent composite material containing a filler.

In Figs. 4A to 4C, the sealing layer 26a is laid with no gap left from the ultrasonic oscillating elements 251. This, however, is not meant as any limitation; the sealing layer 26a may be laid with a gap left from the ultrasonic oscillating elements 251. With this structure, it is possible to suppress or prevent propagation of the ultrasonic wave or vibration generated by the ultrasonic oscillating elements 251 to the LED elements 241. It is thus possible to suppress or prevent breakage of wiring members (unillustrated) wire-bonded to the LED elements 241.

Next, the image generator 30 will be described. The image generator 30 processes the signal detected by the ultrasonic probe 20 to turn it into an image. For example, the image generator 30 generates an optoacoustic image based on a detection signal of an optoacoustic wave, and generates an ultrasonic imaging based on a detection signal of an ultrasonic wave. As shown in Fig. 2, the image generator 30 includes a reception circuit 301, an A/D converter 302, a reception memory 303, a data processor 304, an optoacoustic image reconstructor 305, a discriminator/logarithmic converter 306, an optoacoustic image constructor 307, an ultrasonic image reconstructor 308, a discriminator/logarithmic converter 309, an ultrasonic image constructor 310, an image merger 311, a controller 312, a transmission control circuit 313, and an operation panel 314.

The reception circuit 301 selects, out of the plurality of ultrasonic oscillating elements 251, a part of them, and amplifies a voltage signal (detection signal) with respect to the selected ultrasonic oscillating elements 251. In optoacoustic imaging, for example, the plurality of ultrasonic oscillating elements 251 are divided into two regions adjoining in the Y direction. Of these two regions, one is selected for first-time irradiation, and the other is selected for second-time irradiation. In ultrasonic imaging, for example, an ultrasonic wave is generated while switching is performed from one part of the plurality of ultrasonic oscillating elements 251 to another, that is, from one group of adjoining ultrasonic oscillating elements 251 to another (so-called linear electronic scanning). Accordingly, the reception circuit 301 selects one part after another of the ultrasonic oscillating elements 251 while switching from one group to another.

The A/D converter 302 converts the amplified detection signal from the reception circuit 301 into a digital signal. The reception memory 303 stores the digital signal from the A/D converter 302. The data processor 304 branches the signal stored in the reception memory 303 between the optoacoustic image reconstructor 305 and the ultrasonic image reconstructor 308.

The optoacoustic image reconstructor 305 performs phase matching addition based on the detection signal of an optoacoustic wave, and reconstructs the data of the optoacoustic wave. The discriminator/logarithmic converter 306 performs logarithmic compression and envelope discrimination on the data of the reconstructed optoacoustic wave. The optoacoustic image constructor 307 then converts the data resulting from the processing by the discriminator/logarithmic converter 306 into pixel-by-pixel luminance value data. Thus, optoacoustic image data (e.g., grayscale data) is generated which is composed of the luminance data of every pixel on an X-Y plane. The optoacoustic image reconstructor 305, the discriminator/logarithmic converter 306, the optoacoustic image constructor 307, and the image merger 311 are an example of a cross-sectional image generator and an image former according to the present invention, and together perform the imaging of an optoacoustic wave.

The optoacoustic image data is data that represents the optoacoustic image formed by applying additive averaging to the cross-sectional image of the optoacoustic wave generated in an X-Y plane region inside the tested object 150 right under the ultrasonic probe 20. The optoacoustic image has a pixel size of, for example, 2048 pixels vertically by 128 pixels horizontally. Each pixel is represented by the detection level, on a 256-level grayscale, of the optoacoustic wave after additive averaging. The horizontal (lateral) dimension of the optoacoustic image corresponds to the width (distance in the Y direction) of the cross-sectional image, and the vertical (longitudinal) dimension of the optoacoustic image corresponds to the detection depth (distance in the X direction) relative to the surface of the tested object 150. The size of the cross-sectional image (i.e., the actual size in the X-Y plane region) is, for example, (Width) x (Detection Depth) = 5 centimeters x 5 centimeters. The amount of data of the cross-sectional image generated from the detection signal of the optoacoustic wave generated by single-time irradiation is, for example, 32 megabytes.

On the other hand, the ultrasonic image reconstructor 308 performs phase matching addition based on the detection signal of an ultrasonic wave, and reconstructs the data of the ultrasonic wave. The discriminator/logarithmic converter 309 performs logarithmic compression and envelope discrimination on the data of the reconstructed ultrasonic wave. The ultrasonic image constructor 310 then converts the data resulting from the processing by the discriminator/logarithmic converter 309 into pixel-by-pixel luminance value data. Thus, ultrasonic image data (e.g., grayscale data) is generated which is composed of the luminance data of every pixel on an X-Y plane.

The image merger 311 merges the optoacoustic image data and the ultrasonic image data together to generate merged imaged data. The image merging here may be achieved by superimposing the optoacoustic image on the ultrasonic imaging, or by putting the optoacoustic image and the ultrasonic imaging together side by side (or one on top of the other). The image display 40 displays an image based on the merged imaged data generated by the image merger 311. The image merger 311 may instead output the optoacoustic image or the ultrasonic imaging as it is to the image display 40.

The controller 312 controls the components of the optoacoustic imaging device 100 according to programs and control information stored in an unillustrated non-volatile storage medium (memory). For example, the controller 312 transmits a light trigger signal to the light source driver 22. In a case where the light source 24 includes a plurality of kinds of light sources that emit pulse light of different wavelengths, the controller 312 outputs a wavelength control signal to the light source driver 22. In this case, the light source drive circuit 22a selectively drives the kind of LED light sources which corresponds to the wavelength control signal.

In response to an instruction from the controller 312, the transmission control circuit 313 transmits a drive signal to the acoustoelectric converter 25 to make it generate an ultrasonic wave. The controller 312 also controls the reception circuit 301, etc.

The operation panel 314 accepts input operations by a user, and outputs input signals corresponding to them to the controller 312.

Next, the image display 40 will be described. The image display 40 is a display device furnished with a touch panel, and includes an LCD (liquid crystal display) 401 and an input detector 402. The LCD 401 displays an image (e.g., optoacoustic image) based on the image signal generated by the image generator 30. The input detector 402 accepts input operations by the user. In response to touch operations by a user with a finger, a touch pen, or the like, the input detector 402 outputs input signals corresponding to them to the controller 312.

### Second Embodiment

Next, a second embodiment of the present invention will be described. The following description focuses on structural differences from the first embodiment. Such parts as find their counterparts in the first embodiment are identified by common reference signs, and no overlapping description will be repeated unless necessary.

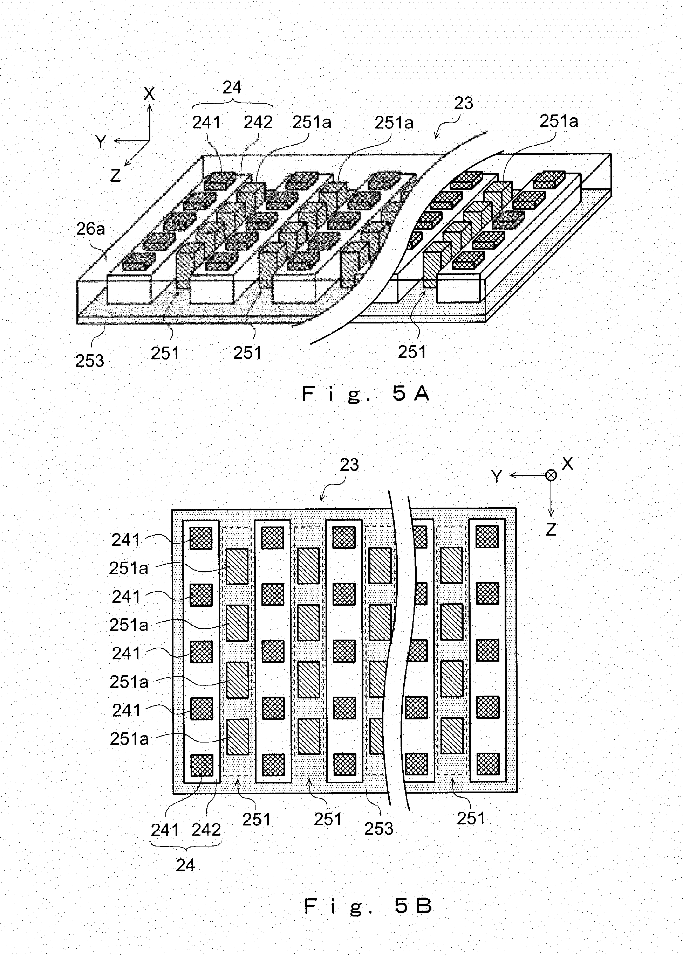

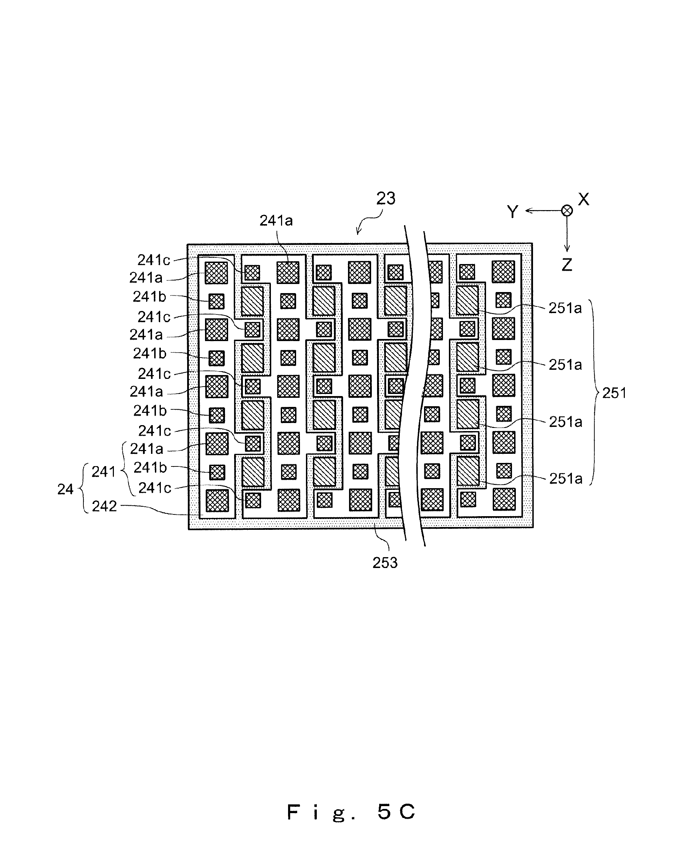

Fig. 5A is a schematic perspective view showing an example of the structure of the optoacoustic module 23 according to the second embodiment. Fig. 5B is a schematic top view showing an example of the structure of the optoacoustic module 23 according to the second embodiment. In Figs. 5A and 5B, for the sake of easy understanding of the structure of the optoacoustic module 23, the irradiating direction and the ultrasonic wave detecting surface are shown to point upward, and the acoustic lens 252 is omitted from illustration. In Figs. 5A and 5B, the sealing layer 26a is laid with no gap left from the ultrasonic oscillating elements 251. This, however, is not meant as any limitation; the sealing layer 26a may be laid with a gap left from the ultrasonic oscillating elements 251. The same applies to Fig. 5C, which will be referred to later.

In the optoacoustic module 23 shown in Figs. 5A and 5B, the ultrasonic oscillating elements 251 are each composed of four divided oscillating elements 251a arranged on the backing member 253. The number of divided oscillating elements 251a into which each oscillating element 251 is divided is not limited to the specific number shown in Figs. 5A and 5b, but may instead be two, three, or five or more.

While the ultrasonic oscillating elements 251 are arranged in a first array direction (the Y direction), in each oscillating element 251, the four divided oscillating elements 251a are arrayed in a second array direction (the Z direction) which crosses the first array direction. Accordingly, in Fig. 5B, the divided oscillating elements 251a are arrayed two-dimensionally, for example, in four columns in the first array direction and 128 rows in the second array direction, on a principal surface of the backing member 253.

Moreover, as seen in a plan view from the X direction (e.g., as in Fig. 5B), the LED elements 241 are arranged so as to alternate with divided oscillating elements 251 a in the second array direction in which the four divided oscillating elements 251 a of each oscillating element 251 are arranged.

With this arrangement of the LED elements 241 and the divided oscillating elements 251a, it is possible to irradiate the tested object 150 with light from between the divided oscillating elements 251a in the second array direction. It is thus possible to increase the area irradiated with light on the surface of the tested object 150. It is thereby possible to increase the amount of light with which the tested object 150 is irradiated, and thus to intensify the optoacoustic wave generated inside the tested object 150 (in particular, at the light absorber P). Hence, it is possible to increase the detection intensity of the optoacoustic wave. It is also possible to irradiate a close region right under the ultrasonic oscillating elements 251 with light. Thus, it is possible to intensify the optoacoustic wave generated in a region close to the surface of the tested object 150. It is thus possible to acquire sharp cross-sectional image information, in particular, in a region close to the surface of the tested object 150.

In each ultrasonic oscillating element 251, the four divided oscillating elements 251a can be operated as a single detecting element or as individual divided detecting elements. For example, in a case where they are operated as a single detecting element, a two-dimensional cross-sectional image can be obtained in which the detection signal of an optoacoustic wave output from the four divided oscillating elements 251a has been processed as the detection signal of a single oscillating element 251.

On the other hand, in a case where the divided oscillating elements 251 a are operated as individual divided detecting elements, it is possible to obtain a three-dimensional cross-sectional image according to the detection signal of the individual divided oscillating elements 251a arranged two-dimensionally as shown in Fig. 5B. The three-dimensional cross-sectional image can be generated by processing individually the detection signal of an optoacoustic wave output from the four divided oscillating elements 251a. For example, from the divided oscillating elements 251a arranged two-dimensionally, first, two-dimensional cross-sectional image data on an X-Y plane generated based on the detection signal output from the 128 divided oscillating elements 251 a arranged in the first array direction (Y direction) is acquired; then, it is merged with two-dimensional cross-sectional image information acquired according to the number of divided oscillating elements 251a arranged in the second array direction (Z direction); thereby, a three-dimensional cross-sectional image is obtained.

**Modified Example of the Second Embodiment:** The structure shown in Figs. 5A and 5B is not meant as any limitation; some of the LED elements 241 may be arranged, as seen in a plan view from the X direction, between other LED elements 241 that are adjacent to each other in at least one of the first and second array direction.

Fig. 5C is a schematic top view showing another example of the structure of the optoacoustic module according to the second embodiment. For example as shown in Fig. 5C, LED elements 241 b may be arranged between LED elements 241 a that are adjacent to each other in the Z direction, or LED elements 241c may be arranged between LED elements 241 a that are adjacent to each other in the Y direction.

With such a structure, it is possible to further increase the amount of light with which the tested object 150 is irradiated. Thus, it is possible to further intensify the optoacoustic wave generated inside the tested object 150 (in particular, at the light absorber P). It is also possible to further intensify the optoacoustic wave generated in a region close to the surface of the tested object 150. It is thus possible to generate sharper cross-sectional image information including a region close to the tested object 150.

### Third Embodiment

Next, a third embodiment of the present invention will be described. The following description focuses on structural differences from the first and second embodiments. Such parts as find their counterparts in the first and second embodiments are identified by common reference signs, and no overlapping description will be repeated unless necessary.

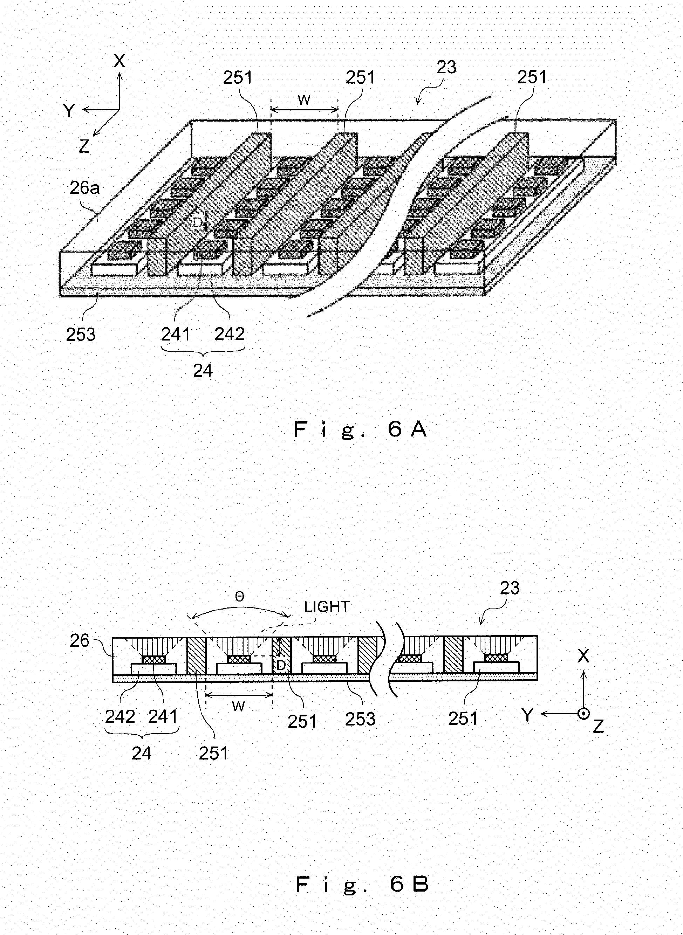

Fig. 6A is a schematic perspective view showing an example of the structure of the optoacoustic module 23 according to the third embodiment. Fig. 6B is a schematic side view showing an example of the structure of the optoacoustic module 23 according to the third embodiment. In Figs. 6A and 6B, for the sake of easy understanding of the structure of the optoacoustic module 23, the irradiating direction and the ultrasonic wave detecting surface are shown to point upward, and the acoustic lens 252 is omitted from illustration. In Figs. 6A and 6B, the sealing layer 26a is laid with no gap left from the ultrasonic oscillating elements 251. This, however, is not meant as any limitation; the sealing layer 26a may be laid with a gap left from the ultrasonic oscillating elements 251.

In Figs. 6A and 6B, D represents the difference in height (shortest distance) between the top surface of the LED elements 241 (the light emission position) and the top surface of the ultrasonic oscillating elements 251 in the light emission direction of the LED elements 241. W represents the interval (shortest distance) between adjacent ultrasonic oscillating elements 251, and θ represents the beam spread angle of the LED elements 241.

In the optoacoustic module 23 shown in Figs. 6A and 6B, with respect to the light emission direction of the LED elements 241, the light emission position of the LED elements 241 is located below the top surface of the ultrasonic oscillating elements 251. The interval W between adjacent ultrasonic oscillating elements 251 is set according to the beam spread angle θ of the LED elements 241 and the difference D in height such that all the light emitted from the LED elements 241 with the beam spread angle θ shines on the tested object 150 without being intercepted by the ultrasonic oscillating elements 251.

In this way, it is possible to locate the light emission position of the LED elements 241 such that the light emitted from the LED elements 241 shines on the tested object 150 without being intercepted by the ultrasonic oscillating elements 251. This enhances the efficiency of use of the emitted light, and it is thus possible to further intensify the optoacoustic wave generated inside the tested object 150, and thereby to increase the detection intensity of the optoacoustic wave. It is also possible to irradiate the tested object 150 with the light emitted from the LED elements 241 with a comparatively wide beam spread angle θ. It is thus possible to further intensify the optoacoustic wave generated in a region close to the surface of the tested object 150, and to acquire sharper cross-sectional image information in a region close to the surface of the tested object 150.

### Fourth Embodiment

Next, a fourth embodiment of the present invention will be described. The following description focuses on structural differences from the first to third embodiments. Such parts as find their counterparts in the first to third embodiments are identified by common reference signs, and no overlapping description will be repeated unless necessary.

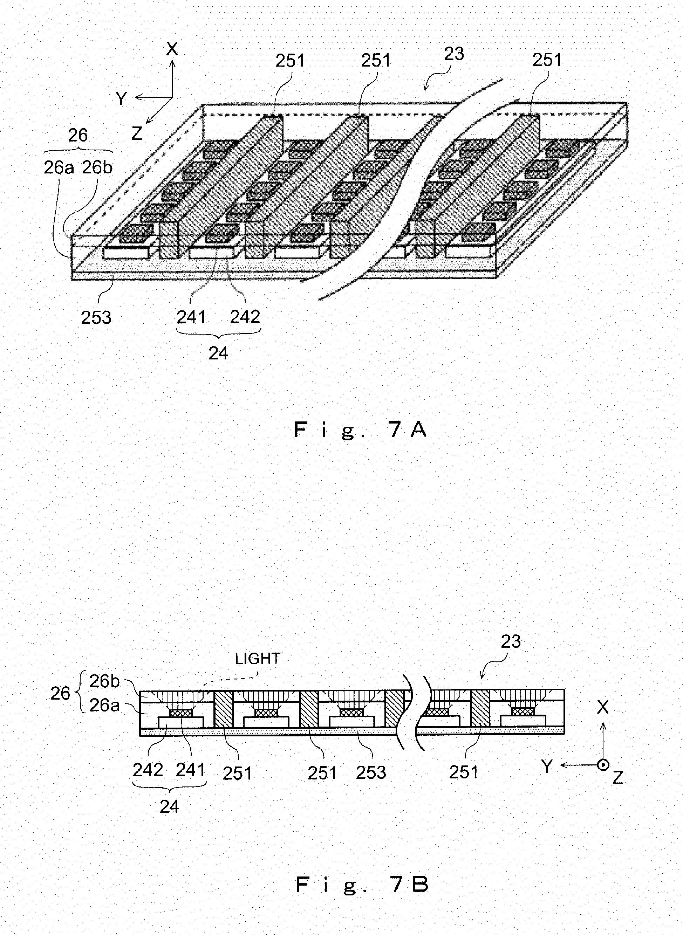

Fig. 7A is a schematic perspective view showing an example of the structure of the optoacoustic module 23 according to the fourth embodiment. Fig. 7B is a schematic side view showing an example of the structure of the optoacoustic module 23 according to the fourth embodiment. In Figs. 7A and 7B, for the sake of easy understanding of the structure of the optoacoustic module 23, the irradiating direction and the ultrasonic wave detecting surface are shown to point upward, and the acoustic lens 252 is omitted from illustration. In Figs. 7A and 7B, the sealing layer 26a and a filling layer 26b are laid with no gap left from the ultrasonic oscillating elements 251. This, however, is not meant as any limitation; at least one of the sealing layer 26a and the filling layer 26b may be laid with a gap left from the ultrasonic oscillating elements 251.

The optoacoustic module 23 shown in Figs. 7A and 7B are configured to include, in addition to the light source 24 and the acoustoelectric converter 25, a transparent or translucent coating 26 provided over the LED elements 241. The coating 26 includes a seal 26a which seals in the LED elements 241 and a filler 26b which is provided over the seal 26a. The filler 26b is formed of a transparent or translucent material that has a Poisson ratio and a refractive index higher than those of the sealing layer 26a and that has a light transmittance of 80% or more. For example, the seal 26a is formed of silicone, and the filler 26b is formed of silicone rubber.

With this structure, the Poisson ratio of the filler 26b, which is provided over the seal 26a, is higher than the Poisson ratio of the seal 26a. As a result, even when vibration of the ultrasonic oscillating elements 251 caused by an elastic wave such as an optoacoustic wave propagates to the coating 26, the vibration is absorbed by the filler 26b, and it is thus possible to suppress or present propagation of the vibration to the seal 26a. Thus, it is possible to suppress or prevent breakage of wiring members wire-bonded to the LED elements 241.

Moreover, the refractive index (n = 1.4 to 1.5) of the filler 26b is higher than the refractive index (n = 1.3 to 1.4) of the seal 26a. It is thus possible to make the light entering the filler 26b through the seal 26a less likely to be totally reflected at the interface between the seal 26a and the filler 26b.

### Fifth Embodiment

Next, a fifth embodiment of the present invention will be described. The following description focuses on structural differences from the first to fourth embodiments. Such parts as find their counterparts in the first to fourth embodiments are identified by common reference signs, and no overlapping description will be repeated unless necessary.

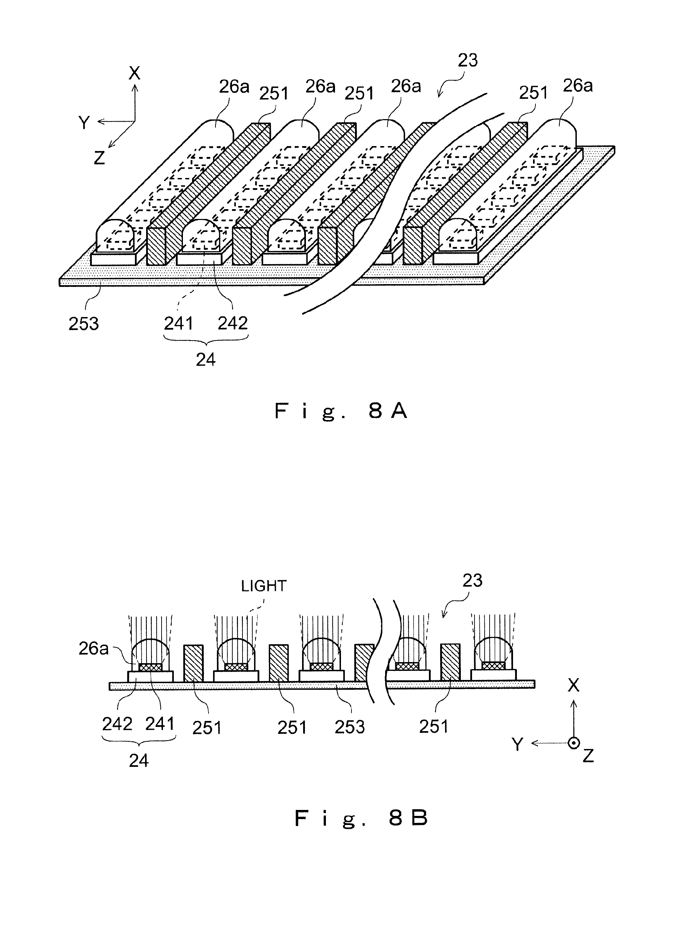

Fig. 8A is a schematic perspective view showing an example of the structure of the optoacoustic module 23 according to the fifth embodiment. Fig. 8B is a schematic side view showing an example of the structure of the optoacoustic module 23 according to the fifth embodiment. In Figs. 8A and 8B, for the sake of easy understanding of the structure of the optoacoustic module 23, the irradiating direction and the ultrasonic wave detecting surface are shown to point upward, and the acoustic lens 252 is omitted from illustration.

The optoacoustic module 23 shown in Figs. 8A and 8B is configured to include a light source 24, an acoustoelectric converter 25, and a transparent or translucent seal 26a. Each seal 26a seals in five LED elements 241 provided on one substrate 242, and has an X-Y section shaped like that of a convex lens like, for example, a cylindrical lens or a toric lens. Accordingly, the seal 26a acts as a converging lens that converges, on a plane parallel to the X-Y section, the light emitted from the LED elements 241 toward the light emission direction. With this structure, the light emitted from the LED elements 241 is converted, and thus, even when the light absorber P is located deep inside the tested object 150, it is possible to irradiate the light absorber P with sufficient light to generate an optoacoustic wave sufficiently intense to generate a cross-sectional image.

Although in Figs. 8A and 8B, the seal 26a seals in five LED elements 241 arrayed in the Z direction, this is not meant as any limitation; instead, for example, separate seals 26a may seal in different LED elements 241 individually. The seal 26a may be formed in the shape of a convex lens that is rotation-symmetric about an axis running in a direction parallel to the X direction. With this structure, it is possible to converge the light emitted from the LED elements 241 more toward a direction parallel to the X direction. It is thus possible to further enhance the efficiency of irradiation of the tested object 150.

Although in Fig. 8B, the seal 26a is laid with a gap left from the ultrasonic oscillating elements 251, this is not meant as any limitation; the seal 26a may be laid with no gap left from the ultrasonic oscillating elements 251. With this structure, it is possible to extend the convex lens-shaped part of the seal 26a over the interval W between adjacent ultrasonic oscillating elements 251. This makes it easier to converge the light emitted from the LED elements 241, and thus makes it easier to irradiate the light absorber P located deep inside the tested object 150 with light.

It would be understood by a person skilled in the art that the embodiments by way of which the present invention is described hereinbefore are merely illustrative and allow for many modifications with respect to how the individual components and processes are combined together within the scope of the present invention.

Possible modifications are as follows. In the embodiments described above, the light source 24 is configured to include LED elements 241. This, however, is not meant to limit the present invention; the light source 24 needs to at least include a light-emitting element that can emit pulsating light. The light source 24 may be, for example, one that includes a semiconductor laser element (a semiconductor laser element light source) or one that includes an organic light-emitting diode element (an organic light-emitting diode light source). The light source 24 may be one that includes at least one of a light-mitting diode device, a semiconductor laser element, and an organic light-emitting diode element. It is thus possible to make the light source 24 compact. It is also possible to irradiate the tested object 150 with pulsating light having a high emission frequency with a simple structure. Thus, it is possible to form more cross-sectional images per unit time, and thus to acquire an optoacoustic image with higher sharpness and higher definition.

In the first to fifth embodiments, in the optoacoustic module 23, the LED elements 241 are arranged so as to alternate with the ultrasonic oscillating elements 251 in the Y direction on the backing member 253. This, however, is not meant to limit the present invention. The LED elements 241 may instead be arrayed so as to alternate with every two or more of the ultrasonic oscillating elements 251.

In the first to fifth embodiments, in the optoacoustic module 23, five LED elements 241 arranged in a row is arrayed between adjacent ultrasonic oscillating elements 251. This, however, is not meant to limit the present invention. The number of LED elements 241 arranged in a row in the Z direction may be one or any number other than five. A plurality of rows of LED elements 241 may be arranged between adjacent ultrasonic oscillating elements 251.

In the first to fifth embodiments, the ultrasonic probe 20 may further include, at one side or both sides of the optoacoustic module 23, an external light source that irradiates the tested object 150 with light with irradiation timing synchronized with that of the pulsating light from the light source 24. It is thus possible to irradiate the tested object 150 with more intense pulsating light, and thus to acquire an even sharper optoacoustic image.

As described above, according to one aspect of the present invention, an optoacoustic wave detector includes: a light source operable to irradiate a light absorber inside a tested object with light; and an acoustoelectric converter operable to detect an optoacoustic wave generated by the light absorber to generate a detection signal based on a result of detection of the optoacoustic wave. Here, the acoustoelectric converter has a plurality of detecting elements arrayed one-dimensionally, and the light source includes light-emitting elements arranged at least between adjacent ones of the detecting elements (a first configuration).

With the first configuration, in the plurality of detecting elements arrayed one dimensionally, the light-emitting elements are arranged at least between adjacent ones of the detecting elements. Thus, it is possible to simplify the structure, and to enhance the efficiency of use of the light emitted from the light-emitting elements. It is also possible to irradiate the tested object with the light from the light-emitting elements efficiently from a position very close to the detecting elements. Thus, it is possible to generate an intense optoacoustic wave at the light absorber inside the tested object, and to increase the detection intensity of the optoacoustic wave. Hence, even with a light source of a comparatively low output, it is possible to detect an optoacoustic wave sufficiently intense to generate a cross-sectional image.

In the optoacoustic wave detector of the first configuration described above, the detecting elements may each include a plurality of divided detecting elements arrayed in a second array direction crossing a first array direction in which the detecting elements are arrayed, and the light-emitting elements may be arranged so as to alternate with the divided detecting elements in the second array direction (a second configuration).

With the second configuration, it is possible to irradiate the tested object with light from between the divided detecting elements in the second array direction. Thus, it is possible to increase the area irradiated with light on the surface of the tested object. It is thereby possible to increase the amount of light with which the tested object is irradiated, and thus to intensify the optoacoustic wave generated inside the tested object (in particular, at the light absorber). Hence, it is possible to increase the detection intensity of the optoacoustic wave. It is also possible to irradiate even a close region right under the detecting elements with light. Thus, it is possible to intensify the optoacoustic wave generated in a region close to the surface of the tested object. It is thus possible to acquire sharp cross-sectional image information in a region close to the surface of the tested object.

In the optoacoustic wave detector of the first or second configuration described above, the light emission position of the light-emitting elements may be located below the top surface of the detecting elements in the light emission direction of the light-emitting elements (a third configuration).

With the third configuration, it is easy to irradiate a region inside the tested object close to the detecting elements with the light emitted from the light-emitting elements with a comparatively wide beam spread angle. It is thus possible to further increase the intensity of the optoacoustic wave generated at a position close to the detecting elements.

In the optoacoustic wave detector of any of the first to third configurations described above, there may be further provided a transparent or translucent coating provided over the light-emitting elements, in which case the coating may include a seal which seals in the light-emitting elements and a filler which is provided over the seal, and the filler may formed of a material having a higher Poisson ratio than the seal (a fourth configuration).

With the fourth configuration, the Poisson ratio of the filler provided over the seal is higher than that of the seal. Accordingly, even when vibration of the detecting elements caused by an elastic wave such as an optoacoustic wave propagates to the coating, the vibration is absorbed by the filler; it is thus possible to suppress or prevent propagation of the vibration to the seal. Thus, even when wiring members are connected to the light-emitting elements, it is possible to suppress or prevent breakage of the wiring members.

In the optoacoustic wave detector of any of the first to fourth configurations described above, the transparent or translucent filler may be formed of a material having a higher refractive index than the transparent or translucent seal which seals in the light-emitting elements (a fifth configuration).

With the fifth configuration, it is possible to make the light entering the filler through the seal less likely to be totally reflected at the interface between the seal and the filler.

In the optoacoustic wave detector of any of the first to fifth configurations described above, the light emitted from the light-emitting elements may be converged toward their light emission direction by the seal which seals in the light-emitting elements (a sixth configuration).

With the sixth configuration, owing to the light emitted from the light-emitting elements being converged, even when the light absorber is located deep inside the tested object, it is possible to irradiate the tested object with sufficient light to generate an optoacoustic wave sufficiently intense to generate a cross-sectional image.

In the optoacoustic wave detector of any of the first to sixth configurations described above, the light-emitting elements may be light-emitting diode elements (a seventh configuration).

Or, in the optoacoustic wave detector of any of the first to sixth configurations described above, the light-emitting elements may be semiconductor laser elements (an eighth configuration).

Or, in the optoacoustic wave detector of any of the first to sixth configurations described above, the light-emitting elements may be organic light-emitting diode elements (a ninth configuration).

With any of the seventh to ninth configurations, it is possible to make the light source compact, and to irradiate the tested object with pulsating light having a high emission frequency with a simple structure. It is thus possible to generate more cross-sectional images per unit time. Hence, it is possible to acquire an optoacoustic image with higher sharpness and higher definition.

According to another aspect of the present invention, an optoacoustic imaging device includes: the optoacoustic wave detector of any of the first to ninth configurations described above; a cross-sectional image generator operable to generate a cross-sectional image of the tested object based on the detection signal; and an image former operable to form an optoacoustic image based on the cross-sectional image (a tenth configuration).

With the tenth configuration, it is possible to perform optoacoustic imaging by using, as a light source, light-emitting elements arranged at least between adjacent detecting elements. Thus, it is not necessary to provide a device for generating light, and this helps simplify the device configuration. It is also possible to enhance the efficiency of use of the light emitted from the light-emitting elements. Further, it is possible to irradiate the tested object with the light from the light-emitting elements efficiently from a position very close to the detecting elements. Thus, it is possible to generate an intense optoacoustic wave at the light absorber inside the tested object, and to increase the detection intensity of the optoacoustic wave. Hence, even with a light source of a comparatively low output, it is possible to detect an optoacoustic wave sufficiently intense to generate a cross-sectional image, and to form an optoacoustic image based on it.

## Claims

1. An optoacoustic detector comprising:
a light source operable to irradiate a light absorber inside a tested object with light;
and
an acoustoelectric converter operable to detect an optoacoustic wave generated by the light absorber to generate a detection signal based on a result of detection of the optoacoustic wave,
wherein
the acoustoelectric converter has a plurality of detecting elements arrayed one-dimensionally, and
the light source includes light-emitting elements arranged at least between adjacent ones of the detecting elements.

2. The optoacoustic detector according to claim 1, wherein
the detecting elements each include a plurality of divided detecting elements arrayed in a second array direction crossing a first array direction in which the detecting elements are arrayed, and
the light-emitting elements are arranged so as to alternate with the divided detecting elements in the second array direction.

3. The optoacoustic detector according to claim 1 or 2, wherein a light emission position of the light-emitting elements is located below a top surface of the detecting elements in a light emission direction of the light-emitting elements.

4. The optoacoustic detector according to any one of claims 1 to 3, further comprising a transparent or translucent coating provided over the light-emitting elements, wherein
the coating includes a seal which seals in the light-emitting elements and a filler which is provided over the seal, and
the filler is formed of a material having a higher Poisson ratio than the seal.

5. The optoacoustic detector according to any one of claims 1 to 4, wherein the transparent or translucent filler is formed of a material having a higher refractive index than the transparent or translucent seal which seals in the light-emitting elements.

6. The optoacoustic detector according to any one of claims 1 to 5, wherein light emitted from the light-emitting elements is converged toward a light emission direction thereof by the seal which seals in the light-emitting elements.

7. The optoacoustic detector according to any one of claims 1 to 6, wherein the light-emitting elements are light-emitting diode elements.

8. The optoacoustic detector according to any one of claims 1 to 6, wherein the light-emitting elements are semiconductor laser elements.

9. The optoacoustic detector according to any one of claims 1 to 6, wherein the light-emitting elements are organic light-emitting diode elements.

10. An optoacoustic imaging device comprising:
the optoacoustic detector according to any one of claims 1 to 9;
a cross-sectional image generator operable to generate a cross-sectional image of the tested object based on the detection signal; and
an image former operable to form an optoacoustic image based on the cross-sectional image.
